# EUROPEAN PATENT SPECIFICATION

(11) **EP 3 096 841 B1**
(45) Date of publication and mention of the grant of the patent: **11.04.2018**
(21) Application number: 15705731.6
(22) Date of filing: 22.01.2015
(51) Int. Cl.: A61Q 19/00, A61Q 19/10, A61K 8/99, A61K 8/02, A61Q 17/00

(54) **SKIN CARE WIPES COMPRISING ADDED NATURAL ANTIBACTERIAL AGENTS**
HAUTPFLEGEWISCHTÜCHER MIT ZUSÄTZLICHEN NATÜRLICHEN ANTIBAKTERIELLEN WIRKSTOFFEN
LINGETTES DE SOIN DE LA PEAU COMPRENANT DES AGENTS ANTIBACTÉRIENS NATURELS AJOUTÉS

(30) Priority: 24.01.2014 US 201461931175 P
(43) Date of publication of application: 30.11.2016
(73) Proprietor: The Procter & Gamble Company, Cincinnati, OH 45202 (US)
(72) Inventor: WEISMAN, Paul, Thomas, Cincinnati, OH 45202 (US); CHARBONNEAU, Duane, Larry, Cincinnati, OH 45202 (US); HEYSE, Serena, Rae, Cincinnati, OH 45202 (US)
(74) Representative: Mather, Peter Geoffrey
(86) International application number: PCT/US2015/012433
(87) International publication number: WO 2015/112697

(56) References cited:
- EP-A2- 0 414 304
- WO-A1-2009/075884
- DE-A1-102007 054 127
- KR-A- 20130 116 856
- US-A1- 2007 154 459
- US-A1- 2010 068 787
- US-A1- 2011 268 777
- US-B1- 6 277 399

## Description

### FIELD OF THE INVENTION

The present invention relates to a wet wipe product including a fibrous and porous substrate material combined with an aqueous personal care lotion, the lotion including a preservative and an added bacteriophage composition, and methods for making the same. The bacteriophage composition may include one or more bacteriophages that are selected to be effective against one or more respective bacteria strains.

### BACKGROUND OF THE INVENTION

Pathogenic bacteria such as Streptococcus, Escherichia, Salmonella, Listeria, Shigella, Campylobacter, Clostridium, and Staphylococcus, and particularly Streptococcus, Escherichia, Salmonella, and Staphylococcus can colonize on skin or other epithelial tissue. Pathogenic bacteria can result in any of a variety of infections such as dermatitis, diaper rash, and impetigo, commonly caused by bacteria such as *Escherichia coli, Streptococcus pyogenes,* and *Staphylococcus aureus*, in which elderly, infants and pre-school children are particularly susceptible. Other pathogenic conditions include urinary tract infections commonly suffered by female infants and women. Moist skin surfaces, such as skin of infants and young children who wear diapers, may be particularly susceptible to bacterial infection.

Personal skin care wipes such as wet wipes designed, for example, for use in cleaning infants' skin during diaper changes, and for use in a number of other skin cleaning applications, have been manufactured and marketed for a number of years. For example, cleaning an infant's skin during a diaper change is desirable because a diaper typically contains the infant's bodily exudates and may hold them in contact with the skin about the diapered area, prior to the time the diaper is changed. Fecal exudates may contain a number of pathogenic bacteria which, if left on the skin, can directly cause skin infections and urinary tract infections, and can also be spread to the caregiver and/or others. Thus, it is desirable to clean the infant's skin of exudate material and remove and/or destroy as much potentially harmful bacteria as possible.

However, commonly used antibacterial agents such as isopropyl alcohol, various surfactants and other antibacterial agents, may be unacceptably harsh, drying and/or irritating to the skin and mucous membranes, particularly those of an infant, and/or may otherwise be unacceptable for use with infants for various reasons. Other antibacterial agents may be insufficiently effective at concentrations that are deemed safe for use with infants. Thus, it has been a challenge to formulate lotion compositions for skin care wipes, such as baby wipes that can effectively remove both soil and potentially harmful bacteria while not irritating or damaging the skin, which, counterproductively, may make the skin more vulnerable to infection. *See*, *e.g*., US 7,569,530.

### DETAILED DESCRIPTION OF THE INVENTION

Bacteriophages are naturally-occurring biological agents, viral in nature, that infect and replicate within bacteria cells. They are ubiquitous, and believed to be the most abundant and diverse biological entities on earth, naturally existing in seawater, fresh water, soil and generally wherever bacteria are present. Many types of bacteriophages kill their host bacteria cell after infection and replication. Because bacteriophages are absolutely specific to a particular type of host bacteria, however, they do not affect humans or animals.

Skin care wipes within the scope of the present disclosure may include a bacteriophage composition including one or more bacteriophages, wherein the bacteriophage composition is in releasable contact with at least a portion of the skin care wipe. These and other embodiments within the scope of the present disclosure are described in detail herein.

In part, these wipes may be particularly advantageous because the transfer of bacteriophage to a desired surface, such as human epithelial tissue (*e.g*., skin or the lining of a cavity or other structure), presents a challenge in view of the non-motile nature of bacteriophage generally. This is particularly the case when the bacteriophage is incorporated into or applied to a solid material, wherein efficacy may depends in part on transfer of the bacteriophage from a solid material. Because the bacteriophage do not move after application to a solid material (aside from possible Brownian or other motion via externally applied forces), they may only be effective in disrupting or otherwise harming bacterial contamination if live bacteria happens to come into contact with the fixed-position bacteriophage for a sufficient period of time. It is believed that the present inventive skin care wipes, which include the bacteriophage composition, facilitate the transfer of the bacteriophages onto a surface susceptible to bacterial contamination, such as human epithelial tissue, which is sufficient to inactivate the colonized bacteria or inhibit the colonization thereof.

### Wipes

As used herein, the skin care wipe may be referred to as a "wipe". In one embodiment, the wipe may be used to treat a surface that is susceptible to colonization by undesirable bacteria, such as any or a combination of bacteria described herein. In general, the surface that is susceptible to colonization by undesirable bacteria may be epithelial tissue, such as human or animal skin of or about a bodily cavity or other structure, such as the perianal region. The surface may be infant skin or adult skin. The surface may be other epithelial tissue such as urothelial tissue.

The present disclosure relates to a bacteriophage composition in combination with a wipe. The combination may be wet or dry; however, wet wipes may be particularly preferred for skin cleaning applications. The term "wet wipe" describes a moistened or wetted piece of substrate material, such as non-woven substrate material, used to clean the skin. Some currently available wipes are intended for the cleaning the perianal area after defecation or urination. Other wipes are available for the cleansing of the face or other body parts, or inanimate objects, like kitchen countertops. Wet wipes are generally of suitable dimensions to allow for convenient handling while being small enough to be easily disposed of. The substrate material of the wipe may be soft and flexible, and may have a textured surface to enhance cleaning performance and/or provide a pleasant feel to the skin. The material may be a non-woven material, and may be made of synthetic compounds. However, woven materials as well as the use of natural compounds like cotton or cellulose in either woven or nonwoven materials are within the scope of the present disclosure. In addition, synthetic and natural compounds can be combined to make up the woven or nonwoven materials. The texture and material of the wipe may affect the cleaning performance of the wipe. Some exemplary non-woven materials may include fibers formed of material(s) selected from the group consisting of polyolefins, polyesters, celluloses, rayons, polyamides, polyesteramides, polyvinyl alcohols, and combinations thereof. A substrate may be manufactured via any process, such as, but not limited to, spunbonding, spunlace, meltblowing, airlaying, wetlaying, coform, and carding processes, and may have a dry basis weight of between about 25 grams per square meter (gsm) and 75 gsm, or between 30 gsm and 65 gsm. Other substrates may be used with the lotion compositions described below.

"Substrate" refers herein to a material which is primarily two-dimensional (*i.e.,* in an XY plane) and whose thickness (in a Z direction) is relatively small (*i.e.,* 1/10 or less) in comparison to the substrate's length (in an X direction) and width (in a Y direction). Non-limiting examples of substrates include a web, layer or layers or fibrous materials, nonwovens, films and foils such as polymeric films or metallic foils. These materials may be used alone or may include two or more layers joined together. As such, a web is a substrate.

"Nonwoven" refers herein to a fibrous structure made from an assembly of continuous fibers, coextruded fibers, non-continuous fibers and combinations thereof, without weaving or knitting, by processes such as spunbonding, carding, meltblowing, airlaying, wetlaying, coforming, or other such processes known in the art for such purposes.

"Fiber" and/or "Filament" as used herein means an elongate particulate having an apparent length greatly exceeding its apparent width, *i.e*., a length to diameter ratio of at least about 10. For purposes of the present disclosure, a "fiber" is an elongate particulate as described above that exhibits a length of less than 5.08 cm (2 in.) and a "filament" is an elongate particulate as described above that exhibits a length of greater than or equal to 5.08 cm (2 in.).

### Substrate

The substrate may be a nonwoven material. The substrate may be homogeneous or may be layered. If layered, the substrate may include at least two, three, four or five layers. The nonwoven material may include one or more layers of such fibrous assemblies, wherein each layer may include continuous fibers, coextruded fibers, non-continuous fibers and combinations thereof.

The substrate of the present disclosure may include co-formed structure. "Co-formed structure" as used herein means that the structure includes a mixture of at least two different materials wherein at least one of the materials includes a filament, such as a polypropylene filament, and at least one other material, different from the first material, includes a solid additive, such as a fiber and/or a particulate. In one example, a co-formed fibrous structure includes solid additives, such as fibers such as wood pulp fibers, absorbent gel materials, filler particles, particulate spot bonding powders and/or clays, and filaments, such as polypropylene filaments.

The substrate may include synthetic fibers. The synthetic fibers can be any material, such as those selected from the group consisting of polyesters (*e.g*., polyethylene terephthalate), polyolefins, polypropylenes, polyethylenes, polyethers, polyamides, polyesteramides, polyvinylalcohols, polyhydroxyalkanoates, polysaccharides, and combinations thereof. Further, the synthetic fibers may be a single component (*i.e.,* single synthetic material or uniform blend of materials makes up entire fiber), bi-component (*i.e.,* the fiber is divided into two or more discrete regions, the regions each being formed of differing synthetic materials or blends thereof, and may include co-extruded fibers and core and sheath fibers) and combinations thereof. Bicomponent fibers may be used as a component fiber of the structure, and/or they may be present to act as a binder for the other fibers present in the fibrous structure. Any or all of the synthetic fibers may be treated before, during, or after manufacture to change any desired properties of the fibers. The substrate may include hydrophilic fibers, hydrophobic fibers, or a combination thereof.

The substrate may include various percentages of natural and/or synthetic fibers. For example, in some exemplary configurations, the substrate may be formed of 100% synthetic fibers. In another exemplary configuration, the substrate may be formed of natural and synthetic fibers. For example, the substrate may be formed of from about 0% to about 90% natural fibers, with the balance comprising synthetic fibers. The substrate may be formed of 10%, 20%, 30%, 40%, 50%, 60%, 70%, or 80% by weight natural fibers.

The substrate may include filaments. Filaments are typically considered continuous or substantially continuous in nature. Non-limiting examples of filaments include meltblown and/or spunbond filaments. Non-limiting examples of materials that can be spun into filaments include natural polymers, such as starch, starch derivatives, cellulose and cellulose derivatives, hemicellulose, hemicellulose derivatives, chitin, chitosan, polyisoprene (*cis* and *trans*), peptides, polyhydroxyalkanoates, and synthetic polymers including, but not limited to, thermoplastic polymers such as polyesters, nylons, polyolefins such as polypropylene, polyethylene, polyvinyl alcohol and polyvinyl alcohol derivatives, sodium polyacrylate (absorbent gel material), and copolymers of polyolefins such as polyethylene-octene, and biodegradable or compostable thermoplastics such as polylactic acid, polyvinyl alcohol, and polycaprolactone. The filaments may be monocomponent or multicomponent, such as bicomponent filaments.

The substrate may be formed of a plurality of filaments, a plurality of solid additives, such as fibers, and a mixture of filaments and solid additives.

In certain configurations, it may be desirable to have particular combinations of fibers to provide desired characteristics. For example, it may be desirable to have fibers of certain lengths, widths, coarseness or other characteristics combined in certain layers, or separate from each other. The fibers may be of virtually any size and may have an average length from about 1 mm to about 60 mm. Average fiber length refers to the length of the individual fibers if straightened out. The fibers may have an average fiber width of greater than about 5 micrometers. The fibers may have an average fiber width of from about 5 micrometers to about 50 micrometers. The fibers may have a coarseness of greater than about 5 mg/100 m. The fibers may have a coarseness of from about 5 mg/100 m to about 75mg/100 m.

It may be desired that the wipe substrate have a particular pore volume distribution. For purposes herein, it may be desired that a wet wipe be adapted to carry a lotion composition including a bacteriophage composition within pores of the substrate structure, but also express the lotion/bacteriophage composition from the pores when wiping pressure is applied thereto, so that the composition is distributed onto the surface (such as skin) upon wiping. Accordingly, it may be desired that the wipe substrate be selected or manufactured to have a pore volume distribution and/or other features described in, for example, U.S. Pat. No. 7,696,109.

For purposes of transporting and distributing a lotion composition including a bacteriophage composition to the skin, the wipe substrate may be manufactured to have features, and desirable liquid uptake, carrying and release characteristics, such as those disclosed in U.S. Pat. App. Ser. Nos. 13/076,492; 13/236,809; 10/277,614; and 10/223,910. In another example, a co-formed substrate as described in U.S. Pat. No. 8,017,534 may be used.

In some configurations, a substrate may include two outer layers consisting of 100%, by total weight, filaments and an inner layer consisting of 100%, by total weight, fibers.

The substrate may include any suitable amount of filaments and any suitable amount of solid additives. For example, the substrate may include from about 10% to about 70% and/or from about 20% to about 60% and/or from about 30% to about 50% by dry weight of the substrate of filaments and from about 90% to about 30% and/or from about 80% to about 40% and/or from about 70% to about 50% by dry weight of the substrate of solid additives, such as wood pulp fibers. In one example, the substrate includes filaments.

The filaments and solid additives may be present in substrate according to the present disclosure at weight ratios of filaments to solid additives of from at least about 1:1 and/or at least about 1:1.5 and/or at least about 1:2 and/or at least about 1:2.5 and/or at least about 1:3 and/or at least about 1:4 and/or at least about 1:5 and/or at least about 1:7 and/or at least about 1:10.

Non-limiting examples of suitable polypropylenes for making the filaments of the present disclosure are commercially available from Lyondell-Basell and Exxon-Mobil.

Any hydrophobic or non-hydrophilic materials within the substrate, such as polypropylene filaments, may be surface treated and/or melt treated with a hydrophilic modifier. Non-limiting examples of surface treating hydrophilic modifiers include surfactants, such as Triton X-100. Non-limiting examples of melt treating hydrophilic modifiers that are added to the melt, such as the polypropylene melt, prior to spinning filaments, include hydrophilic modifying melt additives such as VW351 and/or S-1416 commercially available from Polyvel, Inc. and Irgasurf commercially available from Ciba. The hydrophilic modifier may be associated with the hydrophobic or non-hydrophilic material at various levels. In one example, the hydrophilic modifier is associated with the hydrophobic or non-hydrophilic material at a level of less than about 20% and/or less than about 15% and/or less than about 10% and/or less than about 5% and/or less than about 3% to about 0% by dry weight of the hydrophobic or non-hydrophilic material.

It may be desired to include hydrophilic fibers or filaments, fibers or filaments formed of hydrophilic material, or fibers or filaments that are treated to render them hydrophilic, in the substrate material. This may make the substrate better capable of absorbing, holding and/or carrying an effective quantity of an aqueous bacteriophage composition, or an aqueous lotion composition containing an aqueous bacteriophage composition, which may then be distributed onto a target surface by wiping motion and pressure that expresses the aqueous composition from the substrate. Most natural fibers such as cellulosic fibers (*e.g*., wood pulp, cotton, hemp and linen), and some synthetic fibers such as rayon, viscose and lyocell fibers, are hydrophilic. As an alternative or in addition to treating non-hydrophilic fibers or filaments to render them hydrophilic, it may be desired that the substrate material be formed of at least 10 percent by weight natural or synthetic hydrophilic fibers, or more preferably, at least 20%, at least 30% or at least 40%. Thus, it may be desirable that the substrate material be formed of, *e.g.,* at least 10%, at least 20%, at least 30% or even at least 40% by weight cellulosic fibers or filaments, or at least 10% by weight synthetic hydrophilic fibers or filaments such as fibers or filaments formed of rayon, viscose or lyocell.

The substrate may include optional additives, each, when present, at individual levels of from about 0% and/or from about 0.01% and/or from about 0.1% and/or from about 1% and/or from about 2% to about 95% and/or to about 80% and/or to about 50% and/or to about 30% and/or to about 20% by dry weight of the substrate. Non-limiting examples of optional additives include permanent wet strength agents, temporary wet strength agents, dry strength agents such as carboxymethylcellulose and/or starch, softening agents, lint reducing agents, opacity increasing agents, wetting agents, odor absorbing agents, temperature indicating agents, color agents, dyes, osmotic materials, microbial growth detection agents, antibacterial agents and mixtures thereof.

The fibers may be circular in cross-section, dog-bone shape, delta (*i.e.,* triangular cross section), trilobal, ribbon, or other shapes typically produced as staple fibers. Likewise, the fibers can be conjugate fibers such as bicomponent fibers. The fibers may be crimped and may have a finish, such as a lubricant, applied. In one example, at least some of fibers or filaments of which the substrate is formed may be trilobal. This provides grooves or channels along the surfaces of the fibers/filaments, which may aid in the flow of aqueous composition into and out of the wipe substrate, for better transfer and distribution thereof onto a surface. Examples are described in U.S. Pat. App. Ser. Nos. 13/236,809; 10/277,614; and 10/223,910.

The substrate materials may also be treated to improve the softness and texture thereof. The substrate may be subjected to various treatments, such as physical treatment, hydro-molding, hydro-embossing, and ring rolling, as described in U.S. Patent No. 5,143,679; structural elongation, as described in U.S. Patent No. 5,518,801; consolidation, as described in U.S. Patent Nos. 5,914,084; 6,114,263; 6,129,801 and 6,383,431; stretch aperturing, as described in U.S. Patent Nos. 5,628,097; 5,658,639; and 5,916,661; differential elongation, as described in U.S. Patent No. 7,037,569, and other solid state formation technologies as described in U.S. Patent No. 7,553,532 and U.S. Patent No. 7,410,683; zone activation, and the like; chemical treatment, such as rendering part or all of the substrate hydrophobic, and/or hydrophilic, and the like; thermal treatment, such as thermal-embossing, softening of fibers by heating, thermal bonding and the like; and combinations thereof.

In some configurations, the surface of the substrate may be essentially flat. In other configurations, the surface of the substrate may optionally contain raised and/or lowered portions. The raised and/or lowered portions can be in the form of logos, indicia, trademarks, geometric patterns, and/or images of the surfaces that the substrate is intended to clean (*i.e.,* infant's body, face, etc.). The raised and/or lowered portions may be randomly arranged on the surface of the substrate or be in a repetitive pattern of some form. Without wishing to be bound by theory, it is believed that a textured substrate may further enable the ease of removal of soils by improving the ability to grip or otherwise lift the soils from the surface during cleansing. Any one of a number of texture elements may be useful in improving the ability to grip or otherwise lift the soil from the surface during cleansing such as continuous hydro-molded elements, hollow molded element, solid molded elements, circles, squares, rectangles, ovals, ellipses, irregular circles, swirls, curly cues, cross hatches, pebbles, lined circles, linked irregular circles, half circles, wavy lines, bubble lines, puzzles, leaves, outlined leaves, plates, connected circles, changing curves, dots, honeycombs, and the like, and combinations thereof. The texture elements may be hollow elements. The texture elements may be connected to each other. The texture elements may overlap each other.

The substrate may have a basis weight between about 15, 30, 40, or 45 grams/m² and about 65, 75, 85, 95, or 100 grams/m². A suitable substrate may be a carded nonwoven comprising a 40/60 blend of viscose fibers and polypropylene fibers having a basis weight of 58 grams/m² as available from Suominen of Tampere, Finland as FIBRELLA® 3160. FIBRELLA® 3160 is a 58 grams/m² nonwoven web comprising 60 %, by total weight, of 1.5 denier polypropylene fibers and 40 %, by total weight, of 1.5 denier viscose fibers. Another suitable material may be FIBRELLA® 3100 which is a 62 grams/m² nonwoven web comprising 50 %, by total weight, of 1.5 denier polypropylene fibers and 50 %, by total weight, of 1.5 denier viscose fibers. In both of these commercially available fibrous webs, the average fiber length is about 38 mm. Another suitable material for use as a substrate may be SAWATEX® 2642 as available from Sandler AG of Schwarzenbach/Salle, Germany. Yet another suitable material for use as a substrate may have a basis weight of from about 50 grams/m² to about 60 grams/m² and have a 20/80 blend of viscose fibers and polypropylene fibers. The substrate may also be a 60/40 blend of pulp and viscose fibers. Exemplary nonwoven substrates are described in U.S. Patent Publication 2012/066852 and U.S. Patent Publication U.S. 2011/244199.

In some exemplary configurations, the substrate may be biodegradable. For example, the substrate could be made from a biodegradable material such as a polyesteramide, or a high wet strength cellulose. In some exemplary configurations, the substrate may be dispersible.

### Lotion Composition

A wet wipe may be moistened or wetted with a lotion composition including a bacteriophage composition of the present disclosure. The lotion composition may be aqueous or aqueous emulsion-based. The pH of the composition may be from about pH 3, 4, or 5 to about pH 7, 7.5, or 8. In some exemplary configurations, the pH may be from about 3.5 to about 4.1. The wipes product as packaged (including the supply of wipes and the added liquid lotion composition contents) may have lotion composition contents constituting at least about 200% of the dry weight of the supply of wipes, more preferably at least about 300%, 400%, 500% or even at least about 600%; or between about 200% and about 600%.

In some exemplary configurations, the lotion composition may include a glucomannan. Without being bound by theory, it is believed that a cleansing composition comprising a glucomannan improves the cleaning performance of a wet wipe. Without wishing to be bound by theory, using a cleansing composition comprising a glucomannan in a wet wipe may increase the adhesive interaction between the soil and the wet wipe above the adhesive interaction between the soil and the surface, thereby allowing the soil to detach from the surface upon wiping. The lotion composition may include from about 0.01 %, by total weight, to about 0.50 %, by total weight, of a glucomannan. The lotion composition may include a glucomannan and one or more synergy enhancing agents. Non-limiting examples of synergy enhancing agents include xanthan gum, carrageenan, alginate, locust bean gum, starch, and gellan gum.

The lotion composition may include from about 0.1 %, by total weight, to about 0.5 %, by total weight, or from about 0.12 %, by total weight, to about 0.18 %, by total weight, of one or more synergy enhancing agents. The ratio of glucomannan to synergy enhancing agent present in the lotion composition may be from about 1:1.5 to about 1:10. Exemplary compositions comprising glucomannan and a synergy enhancing agent are described in U.S. Provisional Patent Application No. 61/758,802.

An exemplary wet wipe may include a lotion composition comprising glucomannan and xanthan gum. Another exemplary wet wipe may include a lotion composition comprising glucomannan, carrageenan, and xanthan gum. In a lotion composition comprising glucomannan, carrageenan, and xanthan gum, the ratio of xanthan gum to glucomannan to carrageenan may be from about 1: 0.02: 0.03 to about 1: 0.33: 0.5.

The peak complex viscosity of a lotion composition comprising a glucomannan and a synergy enhancing agent for use in a wet wipe may be greater than about 0.8 Pascal·seconds (hereinafter "Pa·s"), greater than 2.5 Pa·s, or greater than about 3.0 Pa·s. The peak complex viscosity of a lotion composition for use in a wet wipe may be in the range of about 1.0 Pa·s to about 5.0 Pa·s.

In addition, the lotion composition may include various optional ingredients, such as surfactants, emollients, film-formers, preservatives, pH buffers, rheology modifiers, and various other adjunct ingredients, such as described in U.S. Patent Nos. 7,666,827; 7,005,557; 8,221,774; and U.S. Patent Application Publication No. 2011/0268777. It is to be noted that some ingredient compounds can have a multiple function and that all compounds are not necessarily present in the lotion composition.

### Emollient

The lotion composition may include an emollient. Emollients may (1) hydrate the residues (for example, fecal residues or dried urine residues or menses), thus enhancing their removal from the skin, (2) hydrate the skin, thus reducing its dryness and irritation while improving its flexibility under the wiping movement, (3) reduce the adhesive interaction between the soil and the surface, and (4) protect the skin from later irritation (for example, caused by the friction of an absorbent article) as the emollient is deposited onto the skin and remains at its surface as a thin protective layer.

An emollient may include silicone oils, functionalized silicone oils, hydrocarbon oils, fatty alcohols, fatty alcohol ethers, fatty acids, esters of monobasic and/or dibasic and/or tribasic and/or polybasic carboxylic acids with mono and polyhydric alcohols, polyoxyethylenes, polyoxypropylenes, mixtures of polyoxyethylene and polyoxypropylene ethers of fatty alcohols, and mixtures thereof. The emollients may be either saturated or unsaturated, have an aliphatic character and be straight or branched chained or contain alicyclic or aromatic rings.

In some exemplary configurations, the lotion composition may include a mixture of caprylic/capric triglycerides in combination with Bis-PEG/PPG-16/16 PEG/PPG-16/16 dimethicone known as ABIL® CARE 85, available from Degussa Care Specialties of Hopewell, VA.

Various oil materials may function as emollients, while also providing skin benefits, including improving or maintain the integrity of the skin. For example, the lotion composition may include an omega-6 fatty acid. The lotion composition may include at least about 0.003%, from about 0.003% to about 35%, from about 0.015% to about 25%, or from about 0.06% to about 20%,, by total weight, of the lotion and/or coating composition, of omega-6 fatty acid. Exemplary lotion compositions comprising omega-6 fatty acids are described in U.S. Patent Publication No. 2011/0159074 A1.

The omega-6 fatty acid may be added to the lotion composition as an oil material, such as from a vegetable oil. Therefore, in one exemplary configuration, the lotion composition includes an oil material comprising omega-6 fatty acid. The lotion composition may include from about 0.1% to about 70%, from about 0.5% to about 50%, or from about 2% to about 40%,, by total weight, of the lotion and/or coating composition, of the oil material. The oil material may include at least 3%, from about 3% to about 50%, or from about 5% to about 40%, by total weight, of the oil material, of omega-6 fatty acid.

Non-limiting examples of suitable oil materials include high oleic canola Oil (Brassica campestris, B. napus, B. rapa; characterized by having an oleic fatty acid content greater than 70%, *e.g.,* high oleic canola oil, very high oleic canola oil, or partially hydrogenated canola oil), marula kernel oil (Sclerocarya birrea), palm oil (Elaeis Guineensis Oil), palm olein, palm stearin, palm superolein, pecan oil, pumpkin seed oil, oleic safflower oil (Carthamus Tinctorius; characterized by having an oleic fatty acid content of greater than about 30% and omega-6 fatty acid content of less than about 50%, *e.g*., high oleic safflower oil), sesame oil (Sesamum indicum, S. oreintale), soybean oil (Glycine max, *e.g*., high oleic soybean, low linolenic soybean oil, partially hydrogenated), high oleic sunflower oil (Helianthus annus; characterized by having an oleic content of greater than about 40%, *e.g*., mid oleic sunflower or high oleic sunflower oil), and mixtures thereof. Oleic canola oil, palm oil, sesame oil, high oleic safflower oil, high oleic soybean oil, mid oleic sunflower oil, and high oleic sunflower oil are common plant-bred derived oils and may be also be derived from non-genetically modified organisms (non-GMO).

Non-limiting examples of oil materials are commercially-available from a number of vendors, including Cargill for partially hydrogenated soybean oil (*i.e*., Preference® 110W Soybean Oil or Preference® 300 Hi Stability Soybean Oil), mid oleic sunflower oil (*i.e.,* NuSun® Mid-Oleic Sunflower Oil), high oleic sunflower oil (*i.e*., Clear Valley® High Oleic Sunflower Oil), high oleic canola oil, very high oleic canola, and partially hydrogenated low erucic rapeseed oil (*i.e*., Clear Valley® 65 High Oleic Canola Oil and Clear Valley® 75 High Oleic Canola Oil); Lambert Technology for high oleic canola oil (*i.e*., Oleocal C104); Arch Personal Care for marula kernel oil; Pioneer for high oleic soybean oil (*i.e*., Plenish®); Asoyia for low linolenic soybean oil (*i.e*., Ultra Low Linolenic Soybean Oil®); and Dipasa, Inc. for refined sesame oil.

The oil material can further include a blend of oils, including those described *supra*, as well as additional oil materials. Suitable additional oil materials can include acai berry oil, almond oil, avocado oil, beech oil, brazil nut oil, camelina sativa oil (family Brassicaceae, *e.g*., Camelina Sativa, Gold of Pleasure, False Flax, etc.), camellia seed oil, canola oil, carrot seed oil, cashew nut oil, caster oil, cherry kernel oil, chia oil, corn oil, cottonseed oil, hydrogenated cottonseed oil, evening primrose oil, filbert (hazelnut) oil, grapeseed oil, hemp oil, hickory nut oil, jojoba oil, kukui oil, lanolin, olive oil (Olea europaea), macadamia oil, maringa oil, meadowfoam oil, neem oil, palm kernel oil, olive oil, passionflower oil (family Passiflora, Passiflora Incarnata), peanut oil, peach kernel oil, pistachio nut oil, rapeseed oil, rice bran oil, rose hip oil, safflower oil, sorghum oil, soybean oil, sunflower seed oil, tall oil, vegetable oil, vegetable squalene, walnut oil, wheat germ oil, and mixtures thereof. The oil material of the present disclosure can be selected from the group consisting of camelina sativa seed oil, oleic canola oil, evening primrose oil, marula kernel oil, palm oil, palm olein, palm stearin, palm superolein, passiflora incarnata seed oil, pecan oil, pumpkin seed oil, oleic safflower oil, sesame oil, soybean oil, oleic sunflower oil, vegetable oil, and mixtures thereof.

Suitable, commercially available oil materials include a mixture of vegetable oil and camelina sativa seed oil (commercially-available as Lipex® Omega 3/6 from Aarhus Karlshamn Sweden AB), a mixture of vegetable oil and passiflora incarnata seed oil (commercially-available as Lipex® Omega Passiflora from Aarhus Karlshamn Sweden AB), a mixture of vegetable oil and evening primrose oil (commercially-available as Lipex Omega EPO from Aarhus Karlshamn Sweden AB), high oleic canola oil (commercially-available as Clear Valley® 75 High Oleic Canola Oil from Cargill), and mixtures thereof.

### Surfactant

The lotion composition may include one or more surfactants. The surfactant can be an individual surfactant or a mixture of surfactants. The surfactant may be a polymeric surfactant or a non-polymeric one. The surfactant may aid in emulsification of oily components such as various emollients, and may aid in dissolution and removal of the soils from the surface being cleansed. The surfactant or combinations of surfactants may be mild, which means that the surfactants provide sufficient cleaning or detersive benefits but do not overly dry or otherwise harm or damage the skin. The surfactant, when present in the lotion composition, may be present in an amount ranging from about 0.5 %, 1 %, or 4 %, by total weight, to about 0.001 %, 0.01 % or 0.02 %, by total weight, of the lotion composition. The surfactant may include PEG-40 Hydrogenated Castor Oil, manufactured by Clariant International Ltd. of Switzerland under the designation EMULSOGEN® HCW049.

A wide variety of surfactants are useful herein and include those selected from the group consisting of anionic surfactants, nonionic surfactants, cationic surfactants, amphoteric surfactants, zwitterionic surfactants, and mixtures thereof.

A wide variety of anionic surfactants are useful herein. Non-limiting examples of anionic surfactants include those selected from the group consisting of carboxylates, sarcosinates, sulfates, sulfonates, isethionates, taurates, phosphates, lactylates, glutamates, and mixtures thereof.

Nonionic surfactants useful herein include, but are not limited to, those selected from the group consisting of alkyl glucosides, alkyl polyglucosides, polyhydroxy fatty acid amides, alkoxylated fatty acid esters, alkoxylated fatty alcohol ethers, sucrose esters, and mixtures thereof.

Amphoteric surfactants suitable for use in the present compositions include those surfactants broadly described as derivatives of aliphatic secondary and tertiary amines in which the aliphatic radical can be straight or branched chain and wherein one of the aliphatic substituents contains from about 8 to about 18 carbon atoms and one contains an anionic water solubilizing group such as carboxy, sulfonate, sulfate, phosphate, or phosphonate. Useful amphoteric surfactants include the group consisting of cocoamphoacetate, cocoamphodiacetate, lauroamphoacetate, lauroamphodiacetate, and mixtures thereof.

Zwitterionic surfactants suitable for use herein include those surfactants broadly described as derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one contains an anionic group such as carboxy, sulfonate, sulfate, phosphate or phosphonate. Useful zwitterionic surfactants include betaines, amphoacetates and sulfobetaines, *e.g*., cocoamidopropylbetaine, sodium laurylamphoacetate and cocoamidopropylhydroxysultaine.

### Rheology Modifier

The cleaning composition may include one or more rheology modifiers. A rheology modifier may (1) help to stabilize the lotion composition on a substrate, (2) enhance the transfer of the lotion composition to the skin, and (3) enhance the uniformity of the layer of the lotion composition on the skin. For example, rheology modifiers may help to preserve a homogeneous distribution of the lotion composition within a stack of the substrates. Any composition that is in fluid form may have a tendency to migrate to the lower part of the wipes stack during prolonged storage. This effect may create an upper part of the stack of substrates having less lotion composition than the bottom part of the stack.

Non-limiting examples of rheology modifiers include, but are not limited to, rheology modifiers comprising: polysaccharide units, *e.g*., cellulose, xanthan gum, diutan gum, carrageenan, gellan gum, welan gum, pectin, sclerotium gum, starch, galactoarabinan, alginate, and modified-forms thereof; homopolymers of acrylic acid; acrylic acid cross-linked with a polyfunctional compound, *e.g*., carbomer and acrylate crosspolymer; copolymers of acrylic acid, acrylate esters, maleic acid and the like, generally known as the alkali swellable emulsions (ASE) group; hydrophobically-modified copolymers of acrylic acid, acrylate esters, maleic acid and the like, generally known as the hydrophobically-modified alkali swellable emulsions (HASE) group; polyethylene glycol units of varying length connected by urethane linkages and terminated with hydrophobic end groups, generally known as the hydrophobically-modified ethoxylated urethane resins (HEUR) group; organoclays; silicas; and combinations thereof.

Rheology modifiers, when present in the lotion composition, may be present in the range of about 0.01 %, 0.015 %, or 0.02 %, by total weight, to about 2 %, by total weight, of the lotion composition.

### Preservative

Controlling microbiological growth may be beneficial in water based products such as lotion compositions intended for use in wet wipes. Various bacteria, yeasts and/or molds may colonize and grow in a lotion composition, feeding, for example, on oily or fatty components, and thereby degrading the lotion composition in various ways. Because bacteriophages specifically target particular bacteria, the presence of a bacteriophage composition in the lotion composition cannot be relied upon to prevent the growth of microorganism species other than the target bacteria. However, surprisingly, bacteriophages within the scope of the present disclosure can survive in an environment in which general antimicrobial agents are present, such as a wipes lotion containing a preservative system. Conversely, because a preservative system should be suitably mild so as not to irritate or damage skin upon use, preservatives/preservative systems suitable (and in concentrations suitable) for preserving a lotion composition while in the wipes package may be relatively ineffective at controlling bacteria on the skin after the wipe has been used - unless another agent is included such as a bacteriophage composition.

The lotion composition may include a preservative or a combination of preservatives acting together as a preservative system. Preservatives and preservative systems are used interchangeably in the present disclosure to indicate one unique or a combination of preservative compounds. A preservative may be understood to be a chemical or natural compound or a combination of compounds reducing the growth of microorganisms, thus enabling a longer shelf life for a package of substrates (opened or not opened) as well as creating an environment with reduced growth of microorganisms when transferred to the skin during the wiping process.

The spectrum of activity of the preservative may include bacteria, molds and yeast. Each of such microorganisms may be killed by the preservative. Another mode of action to be contemplated may be the reduction of the growth rate of the microorganisms without active killing. Both actions however result in a drastic reduction of the population of microorganisms.

Materials useful as preservatives include methylol compounds, iodopropynyl compounds, simple aromatic alcohols, paraben compounds, benzyl alcohol, benzoic acid, benzoates, sorbic acid, sorbates, phenoxyethanol, ethxylhexyglycerin, chelators such as ethylenediamine tetraacetic acid, and combinations thereof. Suitable preservative systems are described in U.S. Patent Publication No. 2005/0008680 and U.S. Patent Publication No. 2005/0008681.

Low pH buffering systems, such as a citrate-citric acid buffering system at a pH of less than about 5, may also be employed as part of the preservative system.

In some exemplary configurations, the preservative system may include simple aromatic alcohols (*e.g*., benzyl alcohol). Materials of this type may have effective antibacterial activity. Benzyl alcohol is available from Symrise, Inc. of Teterboro, NJ. In other exemplary configurations, the preservative system may include a mixture of benzyl alcohol, sodium benzoate, phenoxyethanol, ethylhexylglycerin, ethylenediamine tetraacetic acid, citric acid, and sodium citrate dehydrate wherein the pH of the lotion composition is less than about 4. The total concentration of benzyl alcohol may be lower than about 0.4%, by total weight, of the lotion composition. The total concentration of sodium benzoate may be lower than about 0.3%, by total weight, of the lotion composition. The combination of phenoxyethanol and ethylhexylglycerin, which are available as EUXYL® PE 9010 from Schulke & Mayr GmbH of Germany, may be lower than about 0.4%.

In some exemplary configurations, acidic compounds used in sufficient amount to reduce the pH of the lotion composition (*e.g*., pH of less than about 5) may be useful as the preservative, or as a potentiator for other preservative ingredients.

In other exemplary configurations, chelators, such as ethylenediamine tetraacetic acid and its salts, may also be used in preservative systems as a potentiator for other preservative ingredients.

### Adjunct Ingredients

The lotion composition may optionally include other adjunct ingredients. Possible adjunct ingredients may be selected from a wide range of additional ingredients such as texturizers, colorants, soothing agents and medically active ingredients, such as healing actives and skin protectants.

### Bacteriophage Compositions

Wipes within the scope of the present disclosure may include a bacteriophage composition including one or more, two or more, three or more or four or more bacteriophages. For example, the bacteriophage composition may include six bacteriophages, such as LISTSHIELD™(LMP-102™), commercially available from Intralytix, Inc., Baltimore, Maryland. The bacteriophage composition may be blended into a lotion composition. Alternatively, a bacteriophage composition may be applied directly to a wipe substrate material.

As used herein, the term "bacteriophage" (singular) refers to a particular bacteriophage that is effective against one or more bacterial strains. As used in this context, "effective against" means that the referenced bacteriophage infects, lyses, destructs, disrupts, kills, inhibits the growth of, reduces, inactivates or is otherwise effective against to the referenced strain of bacteria. The one or more bacteriophage(s) selected may be lytic, and therefore capable of infecting and killing the target bacteria. As used herein, "bacteria" or "target bacteria" refer to an undesirable microorganism susceptible to infection, lysis, destruction (*e.g*., apoptosis), disruption, death, or inhibited growth, or any alternate mode of cell death caused by a bacteriophage. Different bacteriophages may infect different strains of bacteria with different results, or may infect some strains of bacteria but not others. The target bacteria may be pathogenic (*i.e*., capable of causing infection). However, other bacteria, such as bacteria that may be the source of malodor or other undesirable characteristics, may be target bacterial as well.

The bacteriophages selected may be, independently, wild-type or genetically modified, or any combination thereof. Thus, the one or more of the bacteriophages present in the bacteriophage composition may be wild-type, or all of the bacteriophages present in the bacteriophage composition are wild-type.

One or more of the selected bacteriophages may be a lytic bacteriophage, capable of infection, destruction, and bacterial cell death.

One or more of the selected bacteriophages may be of the taxonomic family selected from the group consisting of Siphoviridae, Podoviridae, Myoviridae, and any combinations thereof. In one example, one or more of the bacteriophages may be of the taxonomic family Myoviridae. In another alternative, all of the selected bacteriophages may be of the taxonomic family Myoviridae.

One or more of the selected bacteriophages may be a lytic bacteriophage of the taxonomic family Myoviridae. When the bacteriophage composition includes two or more bacteriophages, two or more of the bacteriophages may be lytic bacteriophages of the taxonomic family Myoviridae. When the bacteriophage composition includes three or more bacteriophages, three or more of the bacteriophages may be lytic bacteriophages of the taxonomic family Myoviridae.

The bacteriophage is a lytic bacteriophage of the taxonomic family Myoviridae and the taxonomic subfamily Teequatrovirinae. In a particular example, the bacteriophage is a lytic T4 phage. Non-limiting examples may include *Enterobacteria phage T2*, *Enterobacteria phage T4*, *Enterobacteria phage T6, phage JS10*, *phage JS98*, *phage RB51*, and any combination thereof.

The selected bacteriophage(s) may be effective against gram-positive or gram-negative pathogenic bacteria, or a combination thereof.

The selected bacteriophage maybe effective against Enterobacterium such as, for example, Salmonella, Escherichia, or Shigella. Additionally or alternatively, one of more of the selected bacteriophages may be effective against a strain of bacteria of a taxonomic genus selected from the group consisting of Streptococcus, Escherichia, Salmonella, Listeria, Shigella, Campylobacter, Clostridium, Staphylococcus, Pseudomonas, Mycobacterium, and any combinations thereof. One or more of selected the bacteriophages may be effective against a strain of bacteria of a taxonomic genus selected from the group consisting of Streptococcus, Escherichia, Salmonella, Listeria, Staphylococcus, Pseudomonas, and any combinations thereof.

In another alternative, the selected bacteriophage may be effective against Streptococcus, such as, for example, *Streptococcus pyogenes.* The selected bacteriophage may be effective against Escherichia, such as, for example, *Escherichia coli.* The selected bacteriophage may be effective against Salmonella, such as, for example, *Salmonella enteritidis*, *Salmonella typhimurium, Salmonella heidelberg, Salmonella newport, Salmonella hadar,* and any combinations thereof. The selected bacteriophage(s) may be a combination such as, for example, those found in SALMOLYSE™, containing six bacteriophages and commercially available from Intralytics, Inc., Baltimore, Maryland. The selected bacteriophage(s) may be a combination of strains such as, for example, those found in SALMOFRESH™, containing six bacteriophages and commercially available from Intralytix, Inc., Baltimore, Maryland. The selected bacteriophage(s) may be a combination such as, for example, those found in SALMONELEX™, a broad-spectrum combination of bacteriophages commercially available from Micreos B.V., Netherlands. The selected bacteriophage may be effective against Listeria, such as, for example, *Listeria monocytogenes.* The selected bacteriophage(s) may be a combination of strains such as, for example, those found in LISTSHIELD™(LMP-102™), containing six bacteriophages and commercially available from Intralytix, Inc., Baltimore, Maryland. The selected bacteriophage(s) may be a combination such as, for example, those found in LISTEX™ P100, a broad-spectrum combination of bacteriophages commercially available from Micreos B.V. The selected bacteriophage may be effective against Shigella, such as, for example, *Shigella sonnei* or *Shigella flexneri.* The selected bacteriophage may be effective against Campylobacter, such as, for example, *Campylobacter jejuni.* The selected bacteriophage may be effective against Clostridium, such as, for example, *Clostridium botulinum* or *Clostridium difficile*.

The selected bacteriophage may be effective against Staphylococcus, such as, for example, *Staphylococcus aureus*, and in a more particular example, antibiotic-resistant strains thereof such as MRSA and MDRSA which in recent years have become an increasing problem in hospital and pediatric settings.

The selected bacteriophage may be effective against Pseudomonas, such as, for example, *Pseudomonas aeruginosa.* The selected bacteriophage may be effective against Mycobacterium, such as, for example, *Mycobacterium tuberculosis.*

Various methods of isolating and concentrating quantities of specific bacteriophages and producing bacteriophage compositions, or variants thereof, that may be suitable for preparation of a skin care wipe, are further described in, for example, U.S. Pats. Nos. 6,699,701; 7,459,272; 7,745,194; and 8,003,323.

The bacteriophage composition may include at least about 1 x 10² PFU/ml (as is commonly understood in the art, plaque forming units/ml) of the one or more bacteriophages, or from about 1 x 10² PFU/ml to about 1 x 10¹² PFU/ml of the one or more bacteriophages. The wipes lotion may be formulated with bacteriophage composition to include from about 1 x 10² PFU/ml to about 1 x 10¹² PFU/ml of the one or more bacteriophages, or from about 1 x 10³ PFU/ml to about 1 x 10⁶ PFU/ml of the one or more bacteriophages.

The one or more bacteriophages may be applied so as to be present in a concentration of at least about 1 x 10² PFU on any square centimeter of the skin care wipe. In another embodiment herein, from about 1 x 10² PFU to about 1 x 10⁹ PFU of the one or more bacteriophages may be present on any square centimeter of the skin care wipe. In yet another embodiment herein, from about 1 x 10³ PFU to about 1 x 10⁶ PFU of the one or more bacteriophages may be present on any square centimeter of the skin care wipe.

### Protection from UV Radiation

Bacteriophages are prone to have decreased activity or potency upon exposure to sunlight or ultraviolet (UV) radiation. Such exposure may have a damaging effect on the bacteriophage, decreasing lytic ability. Fibrous structures and/or aqueous compositions including bacteriophage(s) might also be susceptible to such decreased bacteriophage activity upon exposure to sunlight or UV radiation. Accordingly, there may be a need for a manufacturer of wet wipes, with an aqueous personal care composition including bacteriophage(s), to devise approaches or means for maintaining the activity of bacteriophage(s) in the aqueous personal care composition during distribution of the fibrous structure from the manufacturer, to the retailer, to the consumer, and finally while being stored before use in the consumer's household.

To address this problem, the wipe product may be packaged in a container that is formed of material that effectively blocks or prevents passage or transmission of UV radiation therethrough, into the interior of the container. In one example, the wipe product may be packaged in a molded tub or film wrap package formed of one or more polymer resins that themselves sufficiently block UV radiation. In another example, polymer resin(s) forming the container may have blended therein or more blocking agents. In a particular example, the polymer resin(s) may have blended therein a blocking agent such as, for example, particulate zinc oxide or titanium dioxide, to a level sufficiently effective to block UV radiation. With respect to such blocking agent(s), "sufficient" means a content level and/or quality effective to prevent passage of UV radiation into the container to an extent that will deactivate more than 50% of the bacteriophage population originally placed within the container during packaging, under normal, average shipping and storage conditions and time for products of the type.

### Methods within the scope of the present disclosure

The present disclosure is further directed to methods of applying one or more bacteriophages to a surface, including the step of contacting the surface with a wipe as described herein. The surface may be any surface that is susceptible to bacterial contamination. To illustrate, the surface may be epithelial tissue of a human or other animal (including, for example, a companion animal such as a dog or cat). A surface susceptible to bacterial contamination may include human skin or the epithelial surface of a cavity or other body structure, such as the perianal area. For example, the epithelial surface may be urothelial tissue (susceptible to, for example, bacterial urinary tract infection) or infant or toddler skin that is in contact with or covered by absorbent articles such as diapers or training pants.
Within the scope of the present disclosure, at least a portion of the one or more bacteriophages is releasably transferred from the wipe to the surface. For example, from about 1 x 10² to about 1 x 10¹⁰ PFU/cm² of the one or more bacteriophages may be releasably transferred from the wipe to the surface. In one embodiment, such transfer may be accomplished wherein the bacteriophage composition is present on a surface of the wipe that may come in contact with the surface that is susceptible to bacterial contamination.

A wipe may include a bacteriophage composition applied to one or both surfaces thereof. A wipe substrate material may have a bacteriophage composition applied thereto during manufacture. Application processes may include any processes suitable for distributing an aqueous fluid onto a substrate, such as spraying, fogging or misting a fluid containing the composition onto the substrate, dipping or immersing the substrate into a fluid containing the composition, rolling, gravure rolling, flexographic printing, brushing, extruding, ink jet printing or slot coating a fluid containing the composition onto the substrate, etc.

A wipe substrate material may undergo application of the bacteriophage to its surface(s) before or after being combined with a lotion composition. The wipe substrate material may have an aqueous bacteriophage composition applied thereto, and then be dried prior to downstream further processing and/or use. Further downstream processing may include combination of the treated wipe substrate with a lotion composition to produce a wet wipe product.

Alternatively, a bacteriophage composition may be added to a lotion composition that is subsequently combined with a wipe substrate or supply of wipe substrate sheets, to form a supply of wet wipes carrying the bacteriophage composition.

### Array of Wipes Products

In one example of application of the present disclosure, two or more distinct but commonly branded (*i.e*., common brand identifier or common source indicator) wipes products may be simultaneously offered for sale and presented in a retail setting as an array of available products. The two or more distinct wipes products may respectively include two or more differing bacteriophage compositions that include bacteriophages that are active against, respectively, two or more differing species or strains of bacteria (such as, for example, any of the species or strains identified herein), or differing combinations thereof. By way of non-limiting example, a first bacteriophage composition including bacteriophages that are active against one or more species of Streptococcus may be included with a first wipes product, and a second bacteriophage composition included bacteriophages that are active against one or more species of Clostridium may be included with a second wipes product. The respective wipes products may be accordingly differentiated by their respective labeling information. They may also respectively include other differentiating product features, such as differences in substrate, differences in scent (imparted by differing added perfume ingredients) and/or differences in lotion composition. They may also be differentiated by features or indications that are specifically intended for use with or by, for example, particular age groups of humans, *e.g*., newborn infants, toddlers, older children, adolescents, young adults, middle-aged adults, and older adults.

In another example, two or more distinct but commonly branded wipes products in an array may differ in that one or more in the array include a bacteriophage composition, while one or more others in the array do not.

### EXAMPLES

### EXAMPLE 1

This example describes the feasibility of transferring bacteriophage from a disposable paper towel onto a solid surface. The example uses an article comprising bacteriophage and a fibrous structure.

### Method

1. Prepare carriers
   a. Dilute fetal bovine serum to 5% (if at 100% add 1 ml to 19 ml sterile water)
   b. Dip 7 sterile glass slides into serum-to replicate organic load found on a surface
   c. Allow to dry at 37 °C for 40 minutes
2. Spray 5 BOUNTY™ paper towels (commercially available from The Procter & Gamble Company, Cincinnati, OH) with SALMOLYSE™ phage cocktail (10⁹ PFU/ml, 10 sprays ∼3 ml) (commercially available from Intralytics, Inc., Baltimore, Maryland).
3. Fold paper towel in half three times
4. Wipe paper towel across the glass slide five times
   a. Complete a total of 5 sample replicates
5. Place slide into 20 ml sterile saline in a 50 ml conical
   a. Vortex 30s
   b. Pass saline through 0.2 um filter and collect filtrate
6. Complete negative control
   a. Wipe slide with untreated paper towel and complete like above
7. Complete positive controls
   a. Add 50 ml of phage directly onto slide
      i. Place slide into 50 ml conical with 20 ml sterile saline
      ii. Vortex
      iii. Filter
8. Complete plaque assay (using *Salmonella Enteritidis* 781)
   a. Complete , 10 fold serial dilutions on all test samples
      -use each dilution
   b. Negative control no diluting needed
   c. Positive controls complete 4, 10 fold serial dilutions
      -assay dilutions 2, 3, 4

**Results:**

| PFU applied | PFU per sq cm | PFU per surface used to wipe |
|---|---|---|
| 9.30E+09 | 1.26E+07 | 1.16E+09 |

| **Sample** | **Dilution** | **Plate Count** | **PFU/slide** | **LOG** |
|---|---|---|---|---|
| Phage Titer | 7 | 31 | 3.10E+09 | 9.4914 |
| Positive Control | 4 | 82 | 1.64E+08 | 8.2148 |
| Negative Control | 0 | 0 | 0.00E+00 | 0.0000 |
| Replicate 1 | 1 | 18 | 3.60E+04 | 4.5563 |
| Replicate 2 | 1 | 153 | 3.06E+05 | 5.4857 |
| Replicate 3 | 1 | 22 | 4.40E+04 | 4.6435 |
| Replicate 4 | 1 | 175 | 3.50E+05 | 5.5441 |
| Replicate 5 | 2 | 30 | 6.00E+05 | 5.7782 |
| | | **Average:** | 2.67E+05 | 5.2015 |

Bacteriophage can be successfully transferred from a paper towel onto a solid surface. Here, about 5 of the possible 8 logs of phage are transferred from the paper towel onto the slide. This testing is completed with paper towel that had not completely dried and carriers that are also partially wet. While paper towels are typically used on moist surfaces, the paper towel itself is initially dry. Starting with a wet paper towel may have an impact on the ability of the phage to transfer. This is explored further in EXAMPLE 2 herein below.

### EXAMPLE 2

EXAMPLE 1 describes the ability of bacteriophage to transfer from a paper towel onto a solid surface. This EXAMPLE 2 describes the impact on the amount of phage transferred to the surface based on whether the paper towel is wet or dry and whether the solid surface that is wiped is wet or dry.

### Method

1. Prepare carriers
   a. Dilute fetal bovine serum to 5%
   b. Dip 22 sterile glass slides into serum-to replicate organic load found on a surface
   c. Allow to dry
2. Spray 20 BOUNTY™ paper towels (commercially available from The Procter & Gamble Company, Cincinnati, Ohio) with T4 phage (10⁹ PFU/ml a 1:10 dil of lysate, titer to confirm concentration)
   a. 10 sprays per paper towel (∼3 ml)
   b. Fold paper towel in half three times
3. With paper towel still wet, wipe paper towel across the DRY glass slide five times
   a. Complete a total of 5 sample replicates
4. Place slide into 20 ml sterile saline in a 50 ml conical
   a. Vortex 30s
   b. Pass saline through 0.2 um filter and collect filtrate
5. Mist 5 carriers with PBS spray to wet (2 sprays)
   a. With paper towel still wet, wipe paper towel across the WET glass slide five times
   b. Complete a total of 5 sample replicates
6. Place slide into 20 ml sterile saline in a 50 ml conical
   a. Vortex 30s
   b. Pass saline through 0.2 um filter and collect filtrate
7. Allow remaining 10 paper towels to dry
8. Wipe DRY paper towel across the DRY glass slide five times
   a. Complete a total of 5 sample replicates
9. Place slide into 20 ml sterile saline in a 50 ml conical
   a. Vortex 30s
   b. Pass saline through 0.2 um filter and collect filtrate
10. Mist 5 carriers with PBS spray to wet (2 sprays)
11. Wipe DRY paper towel across the WET glass slide five times
   a. Complete a total of 5 sample replicates
12. Place slide into 20 ml sterile saline in a 50 ml conical
   a. Vortex 30s
   b. Pass saline through 0.2 um filter and collect filtrate
13. Complete negative control
   a. Wipe 1 carrier with untreated paper towel and complete like above
14. Place slide into 20 ml sterile saline in a 50 ml conical
   a. Vortex 30s
   b. Pass saline through 0.2 um filter and collect filtrate
15. Complete positive control
   a. Add 100 ml of phage directly onto slide (same sample as sprayed onto paper towels)
   b. Apply so that it covers about the same area of the slide as what you wiped
   c. Allow to dry a bit (no obvious puddles)
      i. Place slide into 50 ml conical with 20 ml sterile saline
      ii. Vortex
         iii. Filter
16. Complete plaque assay (using *E. coli*)
   a. Complete 4, 10 fold serial dilutions on all test samples -use each dilution
   b. Negative control no diluting needed
   c. Positive controls complete 4, 10 fold serial dilutions
      -assay dilutions 2, 3, 4

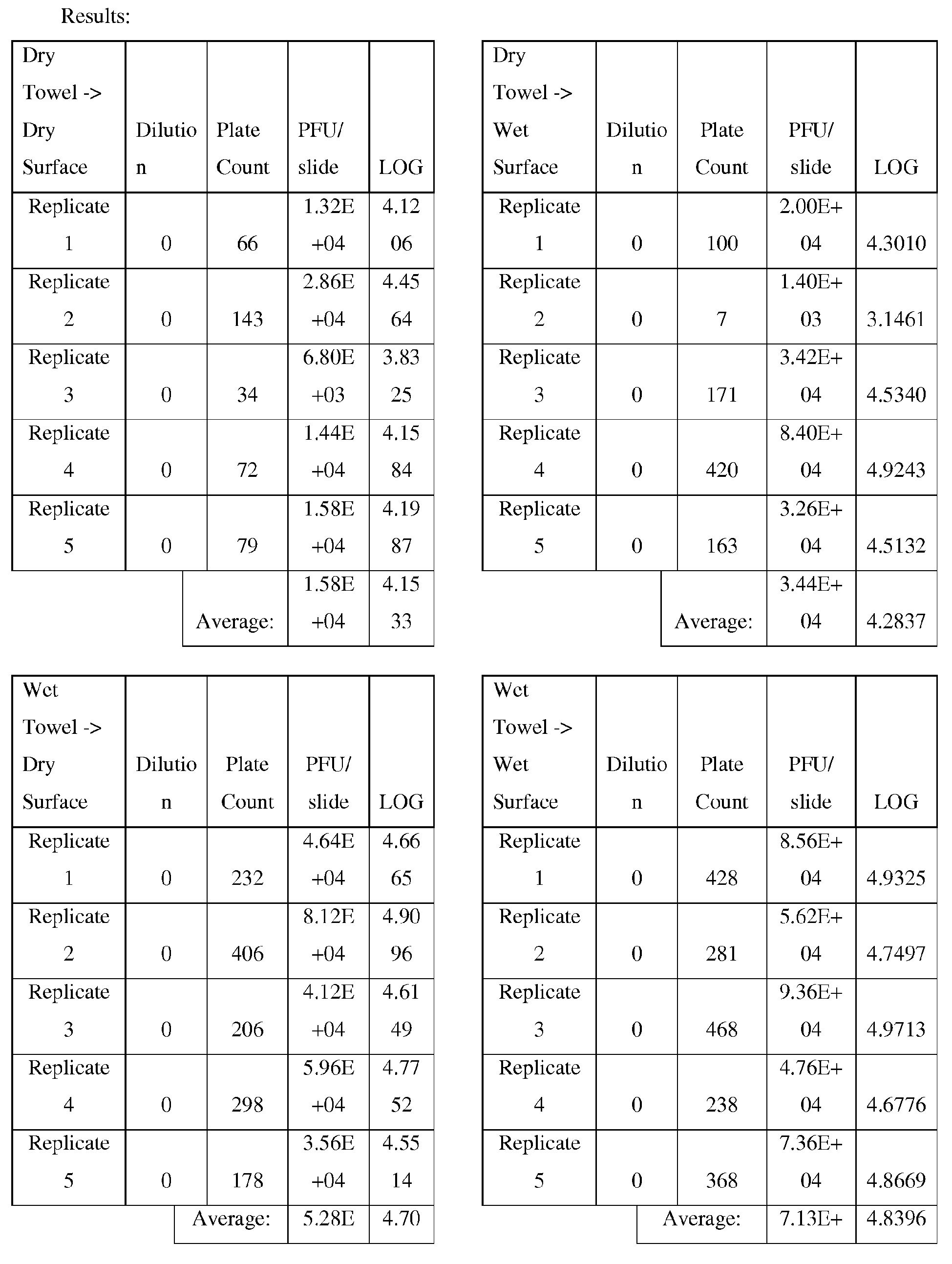

In this EXAMPLE 2, the highest amount of phage transfer from a paper towel to a surface occurs when both the paper towel and the surface are wet. The least amount of transfer is seen when both the paper towel and the surface are dry. Overall, using a dry towel on a wet or dry surface has a similar level of phage transfer. A similar level of phage transfer is also seen when a wet paper towel is used on a wet or dry surface.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A wet wipe product for skin care applications, the wet wipe product comprising:
a fibrous and porous substrate material combined with an aqueous personal care lotion, the aqueous personal care lotion comprising a preservative, wherein the weight content of the personal care lotion in the wet wipe product is at least 200% of the weight of the substrate material prior to combination with the lotion, **characterized in that** the aqueous personal care lotion further comprises an added bacteriophage composition.

2. The wet wipe product of claim 1 wherein the personal care lotion has a bacteriophage concentration of at least 100 PFU/ml.

3. The wet wipe product of either of the preceding claims wherein the aqueous personal care lotion comprises one or more of an emollient, a surfactant and a rheology modifier.

4. The wet wipe product of any of the preceding claims wherein the substrate material comprises a blend of synthetic and natural fibers.

5. The wet wipe product of any of the preceding claims wherein the preservative comprises one or more materials selected from the group consisting of methylol compounds, iodopropynyl compounds, simple aromatic alcohols, paraben compounds, benzyl alcohol, benzoic acid, benzoates, sorbic acid, sorbates, phenoxyethanol, ethxylhexyglycerin, chelators such as ethylenediamine tetraacetic acid, and combinations thereof.

6. The wet wipe product of any of the preceding claims wherein the personal care lotion has a pH of less than about 7, more preferably less than about 6, still more preferably less than about 5, and even more preferably less than about 4.

## Patentansprüche

1. Feuchttuchprodukt für Hautpflegeanwendungen, wobei das Feuchttuchprodukt Folgendes umfasst:
ein faseriges und poröses Substratmaterial, kombiniert mit einer wässrigen Körperpflegelotion, wobei die wässrige Körperpflegelotion einen Konservierungsstoff umfasst, wobei der Gewichtsgehalt der Körperpflegelotion in dem Feuchttuchprodukt wenigstens etwa 200 % des Gewichts des Substratmaterials vor der Kombination mit der Lotion beträgt, **dadurch gekennzeichnet, dass** die wässrige Körperpflegelotion ferner eine hinzugegebene Bakteriophagenzusammensetzung umfasst.

2. Feuchttuchprodukt nach Anspruch 1, wobei die Körperpflegelotion eine Bakteriophagenkonzentration von wenigstens 100 PFU/ml aufweist.

3. Feuchttuchprodukt nach einem der vorstehenden Ansprüche, wobei die wässrige Körperpflegelotion eines oder mehrere eines Erweichungsmittels, eines Tensids und eines Rheologiemodifikators umfasst.

4. Feuchttuchprodukt nach einem der vorstehenden Ansprüche, wobei das Substratmaterial eine Mischung aus synthetischen und natürlichen Fasern umfasst.

5. Feuchttuchprodukt nach einem der vorstehenden Ansprüche, wobei der Konservierungsstoff ein oder mehrere Materialien, ausgewählt aus der Gruppe bestehend aus Methylolverbindungen, lodpropinylverbindungen, einfachen aromatischen Alkoholen, Parabenverbindungen, Benzylalkohol, Benzoesäure, Benzoaten, Sorbinsäure, Sorbaten, Phenoxyethanol, Ethylhexylglycerin, Chelatbildnern wie Ethylendiamintetraessigsäure und Kombinationen davon.

6. Feuchttuchprodukt nach einem der vorstehenden Ansprüche, wobei die Körperpflegelotion einen pH-Wert von weniger als etwa 7, mehr bevorzugt weniger als etwa 6, noch mehr bevorzugt weniger als etwa 5, und noch mehr bevorzugt weniger als etwa 4 aufweist.

## Revendications

1. Produit de type lingette humide pour des applications de soin de la peau, le produit de type lingette humide comprenant :
un matériau de substrat fibreux et poreux combiné avec une lotion de soin personnel aqueuse, la lotion de soin personnel aqueuse comprenant un conservateur, dans lequel la teneur en poids de la lotion de soin personnel dans le produit de type lingette humide est au moins environ 200 % du poids du matériau de substrat avant combinaison avec la lotion, **caractérisé en ce que** la lotion de soin personnel aqueuse comprend en outre une composition bactériophage ajoutée.

2. Produit de type lingette humide selon la revendication 1, dans lequel la lotion de soin personnel a une concentration en bactériophage d'au moins 100 UFP/mL.

3. Produit de type lingette humide selon l'une ou l'autre des revendications précédentes, dans lequel la lotion de soin personnel aqueuse comprend un ou plusieurs parmi un émollient, un agent tensioactif et un agent modifiant la rhéologie.

4. Produit de type lingette humide selon l'une quelconque des revendications précédentes, dans lequel le matériau de substrat comprend un mélange de fibres synthétiques et naturelles.

5. Produit de type lingette humide selon l'une quelconque des revendications précédentes, dans lequel le conservateur comprend un ou plusieurs matériaux choisis dans le groupe constitué de composés de méthylol, composés d'iodopropynyle, alcools aromatiques simples, composés de parahydroxybenzoate, alcool benzylique, acide benzoïque, benzoates, acide sorbique, sorbates, phénoxyéthanol, éthylhexyglycérine, agents chélatants tels que l'acide éthylène-diamine tétra-acétique, et leurs combinaisons.

6. Produit de type lingette humide selon l'une quelconque des revendications précédentes, dans lequel la lotion de soin personnel a un pH inférieur à environ 7, plus préférablement inférieur à environ 6, encore plus préférablement inférieur à environ 5 et même plus préférablement inférieur à environ 4.
